# EUROPEAN PATENT APPLICATION

(11) **EP 4 535 361 A1**
(43) Date of publication of application: **09.04.2025**
(21) Application number: 22943892.4
(22) Date of filing: 31.05.2022
(51) Int. Cl.: G16B 20/20, G16B 40/00, G06N 20/00, C12Q 1/6883

(54) **APPARATUS AND METHOD FOR DETECTING SOMATIC MUTATION BY USING MACHINE LEARNING MODEL CONSTRUCTED REFLECTING DEGREE OF NORMAL CELL CONTAMINATION**

(30) Priority: 26.05.2022 KR 20220064750
(71) Applicant: Seoul National University R & DB Foundation, Seoul 08826 (KR)
(72) Inventor: BAEK, Daehyun, Suwon-si Gyeonggi-do 16507 (KR); JEON, Hyeonseong, Seoul 08806 (KR); AHN, Junhak, Seoul 08754 (KR)
(74) Representative: Laqua, Bernd Christian Kurt
(86) International application number: PCT/KR2022/007718
(87) International publication number: WO 2023/229085

(57) **Abstract**

A somatic mutation detecting apparatus according to the present invention comprises: a memory for storing a program for detecting the somatic mutation; and a processor for executing the program for detecting the somatic mutation, wherein the program for detecting the somatic mutation detects somatic mutation by using a machine learning model, which detects somatic mutation by using, as training data, virtual cancer tissue genome data in which cancer tissue genome data and normal tissue genome data are mixed at different proportions, respectively.

## Description

### Technical Field

The present invention relates to an apparatus and method for detecting a somatic mutation using a machine learning model constructed by reflecting a normal cell contamination level.

### Background Art

Next-generation sequencing technology is a technique that obtains DNA information by cutting DNA obtained from tissue into countless small pieces and decoding the pieces simultaneously. This technique has an advantage of being able to produce significantly more information in the same amount of time compared to capillary electrophoresis sequencing methods such as the existing Sanger sequencing method. Recently, the corresponding technique has developed rapidly, making it possible to obtain relatively accurate genetic information at a low cost. In addition, next-generation sequencing technology is being actively used for personalized treatment along with the field of bioinformatics, which has recently been actively researched.

Various types of software have been designed to detect precise somatic single nucleotide mutations from DNA sequences of cancer patients. Among these, Mutect2 (Cibulskis et al., Nat. Biotech., 2013) and Strelka2 (Fan et al., Genome Biol., 2016) are known representative software. These types of software detect somatic single nucleotide mutations in the DNA sequences of cancer patients based on different mathematical and statistical models. However, these types of software have a limitation in that their accuracy is significantly reduced depending on the normal cell contamination level of a cancer specimen. In particular, since it is almost impossible to collect 100% cancer specimens without collecting normal cells, there is a problem of decreased accuracy. Among existing software, cases where the normal cell contamination level in cancer specimens is considered have only considered in a limited way based on statistical modeling.

The present invention proposes a method of constructing a machine learning model for detecting a somatic mutation using training data by considering various normal cell contamination levels.

### Detailed description of the invention

### Technical Problem

The present invention is intended to solve the above-mentioned problems, and it has a technical problem to provide an apparatus and method for detecting a somatic mutation that can improve the accuracy of somatic mutation detection through training data reflecting various normal cell contamination levels.

However, the technical problem that the present embodiment aims to achieve is not limited to the technical problems described above, and other technical problems may exist.

### Technical Solution

As a technical means for solving the above-described technical problem, the apparatus for detecting a somatic mutation according to a first aspect of the present invention includes a memory configured to store a program for detecting the somatic mutation; and a processor configured to execute the program for detecting the somatic mutation, wherein the program for detecting the somatic mutation detects a somatic mutation by using a machine learning model, which detects a somatic mutation by using, as training data, virtual cancer tissue genome data in which cancer tissue genome data and normal tissue genome data are mixed at different proportions, respectively.

In addition, the method for constructing a machine learning model for detecting a somatic mutation by an apparatus for detecting a somatic mutation according to a second aspect of the present invention includes generating training data based on virtual genome data in which cancer tissue genome data in which a normal cell contamination level is 0% and normal tissue genome data in which a normal cel contamination level is 100% are mixed at different proportions; and (b) constructing a machine learning model that detects a somatic mutation by using the training data.

In addition, the method for detecting a somatic mutation using an apparatus for detecting a somatic mutation according to a third aspect of the present invention includes receiving target genome data for analysis; and inputting the target genome data for analysis into a machine learning model of a program for detecting the somatic mutation to infer a somatic mutation, wherein the machine learning model is constructed based on virtual cancer tissue genome data in which cancer tissue genome data and normal tissue genome data are mixed at different proportions, respectively.

### Advantageous Effects

According to the above-described means of solving the technical problem of the present application, since a learning model is constructed based on training data that actually reflects various levels of normal cell contamination, it can improve the accuracy of somatic mutation detection, unlike conventional somatic mutation detection methodologies where the detection accuracy significantly decreases as the normal cell contamination level in cancer tissue specimens increases.

Since conventionally invented software relies on limited statistical modeling to detect somatic mutations, it has limitations in not properly reflecting the level of normal cell contamination in cancer tissue specimens. This inaccuracy in detecting somatic mutations can lead to incorrect judgments during the treatment of cancer patients, which can be directly linked to the patient's health and life.

The present invention generates training data based on virtual cancer tissue genome data with various levels of normal cell contamination, and it is possible to train the characteristics of actual somatic mutation regions and non-somatic mutation regions in all normal cell contamination situations. Based on this, it guarantees highly accurate somatic mutation detection results and can be applied to precise diagnosis and treatment of cancer patients to provide much improved customized medical services for patients.

### Description of Drawings

FIG. 1 is a block diagram showing the configuration of an apparatus for detecting a somatic mutation according to an embodiment of the present invention.
FIG. 2 is a flowchart showing a method of constructing a machine learning model according to an embodiment of the present invention.
FIG. 3 is a flowchart showing an inference method for a machine learning model according to an embodiment of the present invention.
FIG. 4 is a conceptual diagram showing a method for constructing a machine learning model according to an embodiment of the present invention.
FIG. 5 is a conceptual diagram showing a method of configuring training data according to an embodiment of the present invention.

### Modes of the Invention

Hereinafter, with reference to the attached drawings, embodiments of the present application will be described in detail so that one of ordinary skill in the art to which the present application pertains can easily practice the invention. However, the present application may be implemented in various different forms and is not limited to the embodiments described herein. In order to clearly explain the present application in the drawings, parts that are not related to the description are omitted, and similar reference numerals are assigned to similar parts throughout the specification.

Throughout the present specification, when a part is said to be "connected" to another part, this includes not only a case where it is "directly connected," but also a case where it is "electrically connected" with another element therebetween.

Throughout the specification of the present application, when a member is said to be located "on" another member, this includes not only a case where the member is in contact with the other member, but also a case where another member exists between the two members.

Hereinafter, an embodiment of the present invention will be described in detail with reference to the attached drawings.

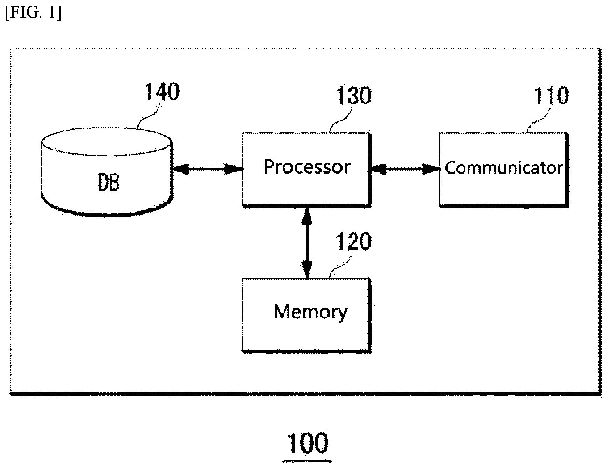

FIG. 1 is a block diagram showing the configuration of an apparatus for detecting a somatic mutation according to an embodiment of the present invention.

When it is described with reference to FIG. 1, the apparatus for detecting a somatic mutation 100 includes a communicator 110, a memory 120, a processor 130 and a database 140.

Next, the communicator 110 receives various genome data for constructing a learning model or genome data of tissue that is the target of somatic mutation detection through an external computing device and the like. The communicator 110 may include a communication module using a wired network such as a Local Area Network (LAN), a Wide Area Network (WAN) or a Value Added Network (VAN), or any type of wireless network such as a mobile radio communication network or satellite communication network. Additionally, the communicator 110 may include modules for communication such as Wi-Fi, Bluetooth communication, infrared communication, ultrasonic communication, Visible Light Communication (VLC), LiFi and the like.

The memory 120 stores a program for detecting the somatic mutation. The program for detecting the somatic mutation is configured to detect somatic mutations by using a machine learning model that detects somatic mutations based on training data in which cancer tissue genome data and normal tissue genome data are mixed at different proportions, respectively. In this case, the machine learning model according to the present invention is constructed by using training data with various mixing proportions of normal tissue genome data to cancer tissue genome data.

Meanwhile, the memory 120 should be interpreted as a general term for non-volatile storage devices that continue to maintain stored information even when power is not supplied and volatile storage devices that require power to maintain stored information. Additionally, the memory 120 may perform a function of temporarily or permanently storing data processed by the processor 130. The memory 120 may include magnetic storage media or flash storage media in addition to volatile storage devices that require power to maintain stored information, but the scope of the present invention is not limited thereto.

The processor 130 executes a program for detecting the somatic mutation stored in the memory 120. The processor 130 may include various types of devices that control and process data. The processor 130 may refer to a data processing device built into hardware that has a physically structured circuit to perform functions expressed by codes or instructions included in a program. In one example, the processor 200 may be implemented in the form of a microprocessor, a central processing unit (CPU), a processor core, a multiprocessor, an application-specific integrated circuit (ASIC), a field programmable gate array (FPGA) and the like, but the scope of the present invention is not limited thereto.

Additionally, the database 140 manages various training data for constructing a learning model for the program for detecting the somatic mutation. For example, it may manage training data with cancer tissue genome data, normal tissue genome data and various mixing ratios thereof. Additionally, the database 140 may manage target genome data for analysis extracted from each subject's tissue, which is input to perform somatic mutation detection using a learning model.

Meanwhile, the apparatus for detecting a somatic mutation 100 may be implemented in the form of various portable terminals in addition to general computing devices. In addition, the apparatus for detecting a somatic mutation 100 may also operate in the form of a server that receives the target genome data for analysis for each subject from an external computing device, inputs the same into the learning model of the program for detecting the somatic mutation, and outputs whether the somatic mutation is detected. In this case, the apparatus for detecting a somatic mutation 100 may operate in a cloud computing service model such as SaaS (Software as a Service), PaaS (Platform as a Service) or IaaS (Infrastructure as a Service). Additionally, the apparatus for detecting a somatic mutation 100 may be constructed in a private cloud, public cloud or hybrid cloud.

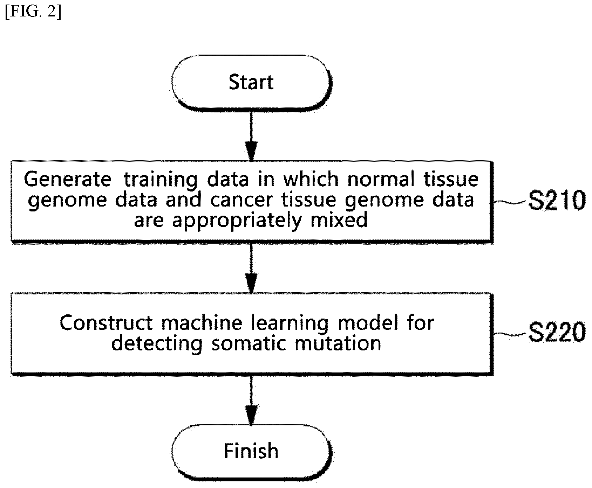

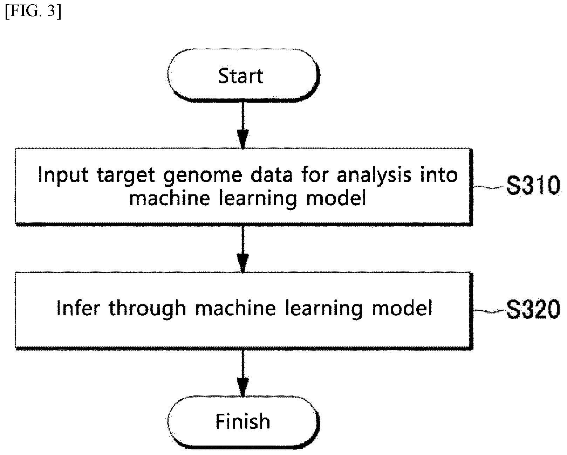

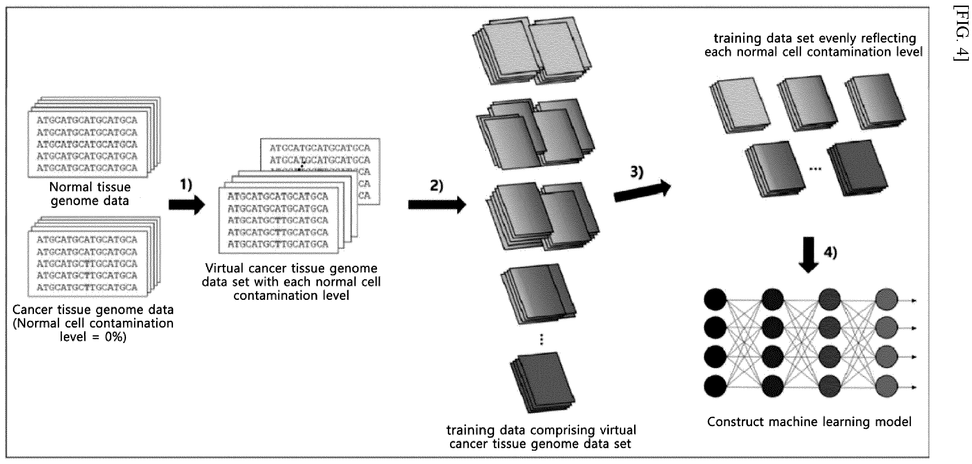

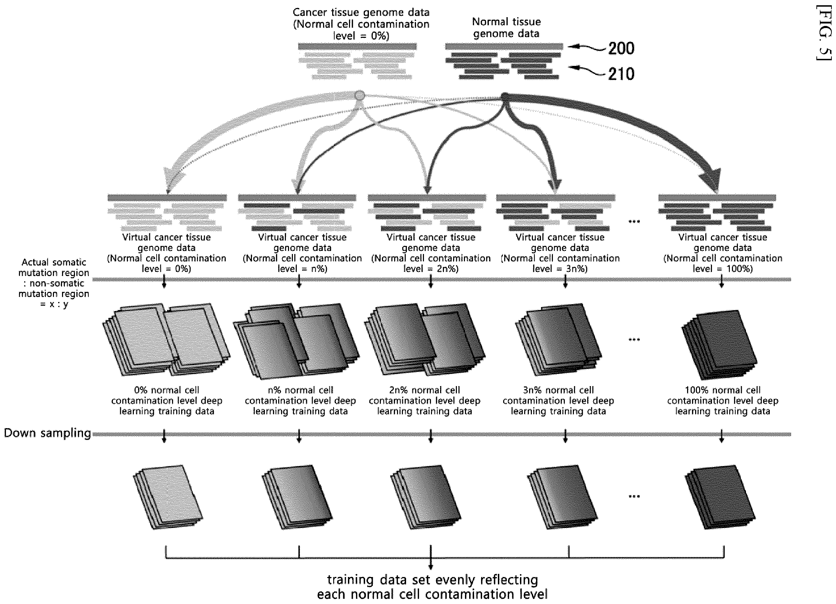

FIG. 2 is a flowchart showing a method of constructing a machine learning model according to an embodiment of the present invention, FIG. 3 is a flowchart showing an inference method for a machine learning model according to an embodiment of the present invention, FIG. 4 is a conceptual diagram showing a method for constructing a machine learning model according to an embodiment of the present invention, and FIG. 5 is a conceptual diagram showing a method of configuring training data according to an embodiment of the present invention.

The method of constructing a machine learning model according to the present invention will be reviewed.

First of all, the apparatus for detecting a somatic mutation 100 generates training data based on virtual cancer tissue genome data in which cancer tissue genome data in which a normal cell contamination level is 0% and normal tissue genome data in which a normal cell contamination level is 100% are mixed at different proportions, respectively S210.

In this case, the normal cell contamination level represents a mixing ratio of normal tissue genome data to cancer tissue genome data. In other words, when no normal tissue is mixed into the cancer tissue genome, the normal cell contamination level is 0%, and the normal cell contamination level increases in proportion to the degree of normal tissue mixing.

In the present invention, multiple virtual cancer tissue genome data are generated in which a normal cell contamination level is between 0% to 100% and a normal cell contamination level is set to be increased uniformly by n% (n is a positive number). For this purpose, the apparatus for detecting a somatic mutation 100 generates virtual cancer tissue genome data in which a normal cell contamination level is m*n% (m is a natural number) by randomly extracting (100-m*n)% of reads without replacement from cancer tissue genome data in which a normal cell contamination level is 0%, and randomly extracting m*n% of reads without replacement from normal tissue genome data in which a normal cell contamination level is 100%.

As shown in FIG. 5, if virtual cancer tissue genome data with a normal cell contamination level of n% is generated, since m is 1, it performs a process in which (100-n)% of reads are randomly extracted without replacement from cancer tissue genome data in which a normal cell contamination is 0%, and n% of reads are randomly extracted without replacement from normal tissue genome data in which a normal cell contamination level is 100%, and then are mixed. Likewise, if virtual cancer tissue genome data with a normal cell contamination level of 2n% is generated, since m is 2, it performs a process in which (100-2n)% of reads are randomly extracted without replacement from cancer tissue genome data in which a normal cell contamination is 0%, and 2n% of reads are randomly extracted without replacement from normal tissue genome data in which a normal cell contamination level is 100%, and then are mixed.

To explain with another example, when generating virtual cancer tissue genome data with a normal cell contamination level of 10%, 90% of reads are randomly extracted without replacement from the cancer tissue genome data with a normal cell contamination of 0%, and 10% of reads are randomly extracted without replacement from the normal tissue genome data, and then are mixed. By using this approach, it is possible to generate virtual cancer tissue genome data with a normal cell contamination level of 10%, where 10% of the total reads are extracted from normal tissue genome data. Likewise, when generating virtual cancer tissue genome data with a normal cell contamination level of 60%, 40% of reads are randomly extracted without replacement from the cancer tissue genome data with a normal cell contamination level of 0%, and 60% of reads are randomly extracted without replacement from the normal tissue genome data, and then are mixed.

For reference, a long bar 200 in the drawing conceptually illustrates the entire genome map, and a short bar 210 shown below conceptually illustrates the genome reads. Through the process of mixing these reads, it is possible to generate virtual cancer tissue genome data mixed with genome data.

Meanwhile, in this way, by using virtual cancer tissue genome data in which cancer tissue genome data and normal tissue genome data are mixed at different proportions, respectively, training data is constructed by equally reflecting the virtual cancer tissue genome data for each contamination level. For example, when there are five normal cell contamination levels to be used for training, 0%, 20%, 40%, 60% and 80%, training data is constructed by randomly extracting each virtual cancer tissue genome data at a ratio corresponding to 1/5 of the total number of training data for each normal cell contamination level. In addition, unlike the virtual cancer tissue genome data for each contamination level equally reflected as above, it is also possible to configure training data in a form where virtual cancer tissue genome data for each contamination level are mixed at different proportions. According to this configuration, various types of learning models may be constructed depending on the intention of a person designing a machine learning model.

Additionally, the form of each training data used in the present invention may be in the form of image data or text data, which may vary depending on the network architecture of a learning model. For example, if a learning model is constructed based on a Convolution Neural Network (CNN)-based architecture, image data is required, and thus, training data is constructed based on images for each genome data. That is, as shown in FIG. 4, training data may be generated using image data of each genome data.

In addition, the generation of this training data is carried out by extracting read information of normal tissue genome data and virtual cancer tissue genome data from actual somatic mutation regions and non-somatic mutation regions, respectively. In other words, read information of normal tissue genome data and cancer tissue genome data is extracted from a somatic mutation region, and read information of normal tissue genome data and cancer tissue genome data is extracted from a non-somatic mutation region.

The determination of an actual somatic mutation region and a non-somatic mutation region may be made through experimental verification based on genome data or by directly modeling the actual somatic mutation region and the non-somatic mutation region using computer simulation. In a somatic mutation training dataset, the ratio of actual somatic mutation regions and non-somatic mutation regions may be set arbitrarily.

Additionally, in order to generate training data, there may be differences in the information extracted from each genome data, but basically, the base information of each read, the quality information of each base, the mapping quality information of reads, the strand information of reads and the distance information from the end of reads may be used. In addition, base information and epigenetic information from a reference genome may be additionally utilized. In this way, in addition to the process of generating virtual cancer tissue genome data, training data is generated using the characteristic information of each genome data for somatic mutation detection, and thus, it is possible to construct a machine learning model to detect somatic mutations based thereon. In other words, the present invention improves the existing learning model that detects somatic mutations based on the characteristic information of genome data, and constructs training data based on virtual cancer tissue genome data with various normal cell contamination levels, and thus, by additionally reflecting a normal cell contamination level that the actual target specimen for analysis inevitably includes, it is possible to detect somatic mutations.

Next, a machine learning model for detecting a somatic mutation is constructed based on the training data generated in this way S220.

As reviewed above, a machine learning model is constructed using training data, but there are no significant restrictions on the learning network architecture used. For example, machine learning models such as linear model, decision tree, random forest, gradient boosting machine (GBM), deep learning model and the like may be used. Additionally, the learning network architecture used in deep learning models also does not have any significant restrictions. For example, it is possible to construct a machine learning model by using deep neural networks such as convolutional neural networks (CNN), recurrent neural networks (RNN), auto encoders, generative adversarial networks (GAN), deep belief networks (DBN) and the like.

CNN may be constructed in a form that includes one or several convolutional layers, pooling layers and fully connected layers. RNN is a deep learning model for training data that changes over time, such as time-series data, and may be configured by connecting networks to a reference time point (t) and the next time point (t+1). Additionally, a long-short term memory (LSTM)-type recurrent neural network may be used.

Next, the method for detecting a somatic mutation using a machine learning model constructed in this way will be reviewed.

Referring to FIG. 3, the apparatus for detecting a somatic mutation 100 receives target genome data for analysis S310.

In this case, the target genome data for analysis is genome data of tissue extracted for tissue examination, and it may be generated by an external computing device.

Next, the target genome data for analysis is input into a machine learning model of the program for detecting the somatic mutation to infer a somatic mutation S320. As reviewed above, the machine learning model is constructed based on virtual cancer tissue genome data in which cancer tissue genome data and normal tissue genome data are mixed at different proportions, respectively.

The method according to an embodiment of the present invention may also be implemented in the form of a recording medium including instructions that are executable by a computer, such as program modules executed by a computer. Computer-readable media may be any available media that can be accessed by a computer, and include both volatile and non-volatile media, removable and non-removable media. Additionally, computer-readable media may include computer storage media. Computer storage media include both volatile and non-volatile, removable and non-removable media implemented in any method or technology for the storage of information such as computer-readable instructions, data structures, program modules or other data.

Although the methods and systems of the present invention have been described with respect to specific embodiments, some or all of the components or operations thereof may be implemented by using a computer system having a general-purpose hardware architecture.

The description of the present application described above is for illustrative purposes, and those skilled in the art will understand that the present application can be easily modified into other specific forms without changing the technical idea or essential features thereof. Therefore, the embodiments described above should be understood in all respects as illustrative and not restrictive. For example, each component described as unitary may be implemented in a distributed manner, and similarly, components described as distributed may also be implemented in a combined form.

The scope of the present application is indicated by the claims described below rather than the detailed description above, and all changes or modified forms derived from the meaning and scope of the claims and their equivalent concepts should be construed as being included in the scope of the present application.

## Claims

1. An apparatus for detecting a somatic mutation, the apparatus comprising:
a memory configured to store a program for detecting the somatic mutation; and
a processor configured to execute the program for detecting the somatic mutation,
wherein the program for detecting the somatic mutation is configured to detect the somatic mutation by using a machine learning model, which detects the somatic mutation by using, as training data, virtual cancer tissue genome data in which cancer tissue genome data and normal tissue genome data are mixed at different proportions, respectively.

2. The apparatus of claim 1, wherein the machine learning model is learned based on multiple virtual cancer tissue genome data in which a normal cell contamination level, which represents a mixing ratio of the normal tissue genome data to the cancer tissue genome data, is between 0% and 100%, and the normal cell contamination level is set to be uniformly increased by n% (n is a positive number).

3. The apparatus of claim 2, wherein the training data comprises each virtual cancer tissue genome data having each normal cell contamination level at equal proportions from each other.

4. The apparatus of claim 2, wherein the machine learning model is trained based on virtual cancer tissue genome data in which a normal cell contamination level is m*n% (m is a natural number) by randomly extracting (100-m*n)% of reads without replacement from cancer tissue genome data in which a normal cell contamination level is 0%, and randomly extracting m*n% of reads without replacement from normal tissue genome data in which a normal cell contamination level is 100%.

5. A method for constructing a machine learning model for detecting a somatic mutation by an apparatus for detecting a somatic mutation, the method comprising:
generating training data based on virtual genome data in which cancer tissue genome data in which a normal cell contamination level is 0% and normal tissue genome data in which a normal cel contamination level is 100% are mixed at different proportions; and
constructing a machine learning model that detects a somatic mutation by using the training data.

6. The method of claim 5, wherein the generating training data comprises generating multiple virtual cancer tissue genome data in which a normal cell contamination level, which represents a mixing ratio of the normal tissue genome data to the cancer tissue genome data, is between 0% and 100%, and the normal cell contamination level is set to be uniformly increased by n% (n is a positive number).

7. The method of claim 6, wherein the training data comprises each virtual cancer tissue genome data having each normal cell contamination level at equal proportions from each other.

8. The method of claim 6, wherein the generating training data comprises generating virtual cancer tissue genome data in which a normal cell contamination level is m*n% (m is a natural number) by randomly extracting (100-m*n)% of reads without replacement from cancer tissue genome data in which a normal cell contamination level is 0%, and randomly extracting m*n% of reads without replacement from normal tissue genome data in which a normal cell contamination level is 100%.

9. A method for detecting a somatic mutation using an apparatus for detecting a somatic mutation, the method comprising:
receiving target genome data for analysis; and
inputting the target genome data for analysis into a machine learning model of a program for detecting the somatic mutation to infer a somatic mutation,
wherein the machine learning model is constructed based on virtual cancer tissue genome data in which cancer tissue genome data and normal tissue genome data are mixed at different proportions, respectively.

10. The method of claim 9, wherein the machine learning model is trained based on multiple virtual cancer tissue genome data in which a normal cell contamination level, which represents a mixing ratio of the normal tissue genome data to the cancer tissue genome data, is between 0% and 100%, and a normal cell contamination level is set to be uniformly increased by n% (n is a positive number).

11. The method of claim 10, wherein the machine learning model is trained based on virtual cancer tissue genome data in which a normal cell contamination level is m*n% (m is a natural number) by randomly extracting (100-m*n)% of reads without replacement from cancer tissue genome data in which a normal cell contamination level is 0%, and randomly extracting m*n% of reads without replacement from normal tissue genome data in which a normal cell contamination level is 100%.
